# EUROPEAN PATENT APPLICATION

(11) **EP 1 834 610 A1**
(43) Date of publication of application: **19.09.2007**
(21) Application number: 07005394.7
(22) Date of filing: 15.03.2007
(51) Int. Cl.: A61F 2/84

(54) **Handle for long self expanding stent**

(30) Priority: 17.03.2006 US 276871
(71) Applicant: Medtronic Vascular, Inc., Santa Rosa, CA 95403 (US)
(72) Inventor: Saint, Sean, San Diego, CA 92129 (US)
(74) Representative: Zimmermann, Gerd Heinrich

(57) **Abstract**

A stent delivery system (200) includes a handle (212) having a housing (214) and a spool (222). A pushrod (204) has a proximal end (204P) connected to the housing (214) of the handle (212). A stent (202) is located near a distal end (204D) of the pushrod (204), wherein the handle (212) has a linear length (Y) less than a linear length (X) of the stent (202). A sheath (206) constrains the stent (202) at a distal end (206D) of the sheath (206). A retraction wire (224) is connected to a proximal end (206P) of the sheath (206) and to the spool (222). Retraction of the sheath (206) is accomplished by winding (coiling) the retraction wire (224) around the spool (222). Accordingly, the handle (212) can be much shorter than the stent (202.

## Description

### BACKGROUND OF THE INVENTION

### FIELD OF THE INVENTION

The present invention relates to an intra-vascular device and method. More particularly, the present invention relates to a device for deployment of a stent for treatment of luminal, i.e., intra-vascular, diseases.

### DESCRIPTION OF RELATED ART

In stent deployment systems, a self-expanding stent was restrained within a sheath. After positioning of the stent at the desired location via fluoroscopic guidance, the physician retracted the sheath to deploy the stent, i.e., to expose the stent and allow it to self-expand. To completely deploy the stent, the physician had to retract the sheath over the entire length of the stent, which was relatively cumbersome and typically required the use of both hands or repeated motion of the physician in the case of long self-expanding stents.

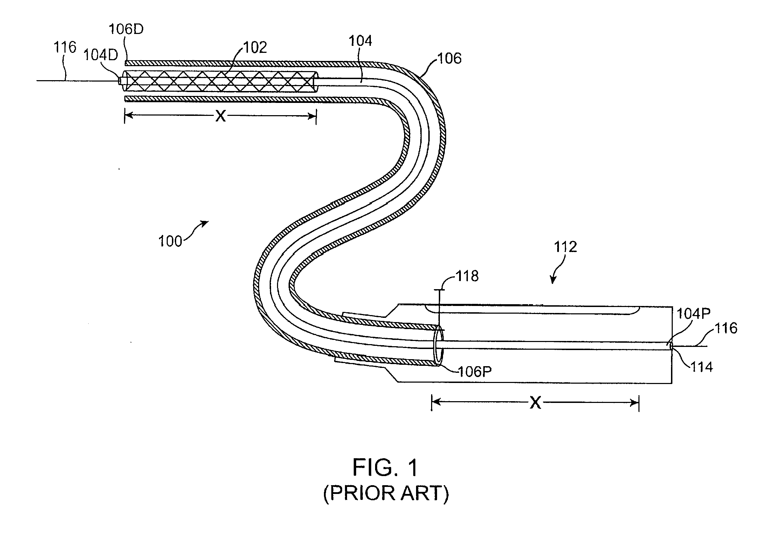

To illustrate, FIG. 1 is a partially cutaway delivery system 100 for deploying a stent 102 in accordance with the prior art. Stent 102 is a radially self-expanding stent.

Delivery system 100 includes a pushrod 104 and a sheath 106, sometimes called a catheter sheath. Pushrod 104 includes a distal end 104D and a proximal end 104P. Stent 102 is placed over distal end 104D of pushrod 104. In one embodiment, distal end 104D further includes radiopaque markers that allow the location of distal end 104D and stent 102 to be precisely tracked. Proximal end 104P of pushrod 104 terminates within and is mounted to a handle 112 or extends through handle 112 and out a port 114 of handle 112.

In this embodiment, pushrod 104 is a hollow tube and includes a guide wire lumen. A guide wire 116 extends through pushrod 104 and extends out distal end 104D. Guide wire 116 further extends through handle 112 and out port 114.

Sheath 106 includes a distal end 106D and a proximal end 106P. Prior to deployment, stent 102 is radially compressed and restrained within distal end 106D of sheath 106. Proximal end 106P of sheath 106 extends into handle 112. Proximal end 106P of sheath 106 is coupled to an actuation button 118, sometimes called a thumb slider, of handle 112. Sheath 106 is a hollow tube and includes a pushrod lumen. Pushrod 104 extends through sheath 106.

During use, stent 102 is placed over distal end 104D of pushrod 104 and is radially compressed and restrained within distal end 106D of sheath 106. Stent 102 is introduced intra-vascularly and guided to the treatment site, e.g., an aneurysm.

Once stent 102 is properly positioned, sheath 106 is retracted by retraction of actuation button 118 thus deploying stent 102. More particularly, stent 102 is self-expandable and as sheath 106 is retracted, stent 102 self-expands and is permanently deployed, e.g., anchored within a lumen of a patient. The guiding of a stent and deployment of a self-expanding stent are well known to those of skill in the art.

During deployment, sheath 106 must move the entire linear length X of stent 102 to completely uncover and thus deploy stent 102. Since actuation button 118 is connected to and moves sheath 106, actuation button 118 must also be moved the linear length X to retract sheath 106 over the entire linear length X of stent 102 as actuation button 118 and sheath 106 move in a strictly linear 1:1 motion.

In the case when stent 102 is a long self-expanding stent, length X is substantial, e.g., 200 mm or more. Accordingly, to accommodate the long travel of actuation button 118, handle 112 must also be very long and at least linear length X. However, long handles are cumbersome and difficult to manipulate.

Therefore, it is an object of the present invention to overcome the problems associated with the prior art at least in part.

### SUMMARY OF THE INVENTION

This object is solved by a stent delivery system according to claims 1, 19, and 20. Further aspects, features, details and advantages of the present application are apparent from the dependent claims, the description, and the accompanying drawings.

In accordance with one example, a stent delivery system includes a handle having a housing and a spool. A pushrod has a proximal end connected to the housing of the handle. A stent is located over a distal end of the pushrod, wherein the handle has a linear length less than a linear length of the stent. A sheath constrains the stent at a distal end of the sheath. A retraction wire is connected to a proximal end of the sheath and to the spool.

Retraction of the sheath is accomplished by winding (coiling) the retraction wire around the spool. Accordingly, the handle can be much shorter than the stent. Illustratively, the handle has a linear length less than the linear length, e.g., 200 mm or more, of the stent. Since the handle is short, the handle is not cumbersome and is easy to manipulate.

The present invention is best understood by reference to the following detailed description when read in conjunction with the accompanying drawings.

### BRIEF DESCRIPTION OF THE DRAWINGS

FIG. 1 is a partially cutaway delivery system for deploying a stent in accordance with the prior art;

FIG. 2 is a partially cutaway delivery system for deploying a stent in accordance with one embodiment of the present invention; and

FIG. 3 is a partially cutaway delivery system having a telescoping strain relief in accordance with one embodiment of the present invention.

Common reference numerals are used throughout the drawings and detailed description to indicate like elements.

### DETAILED DESCRIPTION

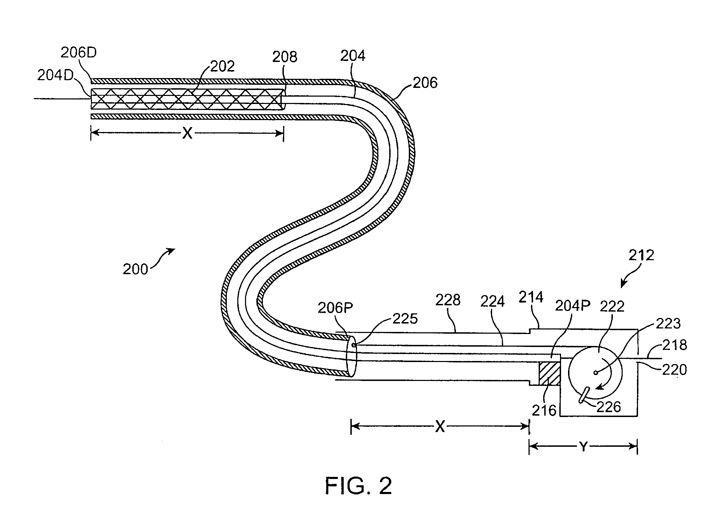

In accordance with one example, a stent delivery system 200 (FIG. 2) includes a handle 212 having a housing 214 and a spool 222. A pushrod 204 has a proximal end 204P connected to housing 214 of handle 212. A stent 202 is shown located over a distal end 204D of pushrod 204 (alternately, the stent could be located beyond the distal end of a pushrod), wherein handle 212 has a linear length Y less than a linear length X of stent 202. A sheath 206 constrains stent 202 at a distal end 206D of sheath 206. A retraction wire 224 is connected to a proximal end 206P of sheath 206 and to spool 222.

Retraction of sheath 206 is accomplished by winding (coiling) retraction wire 224 around spool 222. Accordingly, handle 212 can be much shorter than stent 202. Illustratively, handle 212 has a linear length Y less than linear length X, e.g., 200 mm or more, of stent 202. Since handle 212 is short, handle 212 is not cumbersome and easy to manipulate.

More particularly, FIG. 2 is a partially cutaway delivery system 200 for deploying a stent 202 in accordance with one embodiment of the present invention. Stent 202 is a radially self-expanding stent having a linear length X, e.g., 200 mm or more.

Delivery system 200 includes a pushrod 204 and a sheath 206, sometimes called a catheter sheath. Pushrod 204, sometimes called an inner member or inner shaft, includes a distal end 204D and a proximal end 204P. As is well known, the proximal end of a delivery system is referenced with respect to the operator's handle while the proximal end of a stent is referenced with respect to the end closest to the heart via the length of blood traveled from the heart.

Stent 202 is placed over distal end 204D of pushrod 204. In one embodiment, distal end 204D further includes radiopaque markers that allow the location of distal end 204D and stent 202 to be precisely tracked. Pushrod 204 includes a stop 208 or other structures to prevent stent 202 from being moved proximally during retraction of sheath 206 as discussed further below.

Proximal end 204P of pushrod 204 terminates within and is mounted to a handle 212. More particularly, handle 212 includes a housing 214. Connected to, or integral with, housing 214 is a pushrod anchor 216. Proximal end 204P of pushrod 204 is connected to pushrod anchor 216 and thus to housing 214. Accordingly, pushrod 204 does not move relative to housing 214 of handle 212.

In this embodiment, pushrod 204 is a hollow tube and includes a guide wire lumen. A guide wire 218 extends through pushrod 204 and extends out distal end 204D. Guide wire 218 further extends through handle 212 between a spool 222 and housing 214 and out of a guide wire port 220 of housing 214 of handle 212.

Sheath 206 includes a distal end 206D and a proximal end 206P. Prior to deployment, stent 202 is radially compressed and restrained within distal end 206D of sheath 206. Proximal end 206P of sheath 206 terminates adjacent to handle 212. More particularly, proximal end 206P is located at least a linear length X from pushrod anchor 216 allowing proximal end 206P and thus sheath 206 to be retracted at least linear length X.

Handle 212 further includes spool 222 coupled to housing 214. More particularly, spool 222 rotates on an axle 223 extending through spool 222 and coupled to housing 214.

A retraction wire 224 is connected to and extends between proximal end 206P of sheath 206 and spool 222. Illustratively, retraction wire 224 is glued, welded, screwed to, or otherwise mounted to proximal end 206P of sheath 206 at a bond 225 between retraction wire 224 and proximal end 206P of sheath 206.

Spool 222, sometimes called a coil, provides a means for retracting retraction wire 224 and thus for retracting sheath 206. In this example, spool 222 includes a spool knob 226 that is rotated by the physician thus rotating spool 222 around axle 223. As spool 222 rotates, retraction wire 224 is wound into spool 222 and thus retracted.

Coupled to handle 212 is a strain relief 228. Strain relief 228 relieves strain as it provides a gradual transition among the stiffness of the pushrod 204, sheath 206 and handle 212. In accordance with this example, strain relief 228 extends from handle 212 a distance sufficient to overlap proximal end 206P of sheath 206. More particularly, proximal end 206P of sheath 206 is located inside of strain relief 228.

Sheath 206 is a hollow tube which acts as a pushrod lumen. Pushrod 204 extends through sheath 206.

During use, stent 202 is placed over distal end 204D (or alternately beyond the end, both configurations are considered near the distal end of the pushrod) of pushrod 204 and is radially compressed and restrained within distal end 206D of sheath 206. Stent 202 is introduced intra-vascularly and guided to the treatment site, e.g., a narrowing of an artery.

Once stent 202 is properly positioned, sheath 206 is retracted by rotation of spool 222. For example, spool 222 is rotated around axle 223 by rotation of knob 226 by the physician thus deploying stent 202. More particularly, stent 202 is self-expandable and as sheath 206 is retracted, stent 202 self-expands as it is uncovered and is permanently deployed, e.g., anchored within a lumen of a patient.

During deployment, sheath 206 moves (is retracted) at least the entire length X of stent 202 to completely uncover and thus deploy stent 202. Since retraction of sheath 206 is accomplish by winding (coiling) retraction wire 224 around spool 222, handle 212 can be much shorter than stent 202. Illustratively, handle 212 has a linear length Y less than linear length X of stent 202. Since handle 212 is short, handle 212 is not cumbersome and is easy to manipulate.

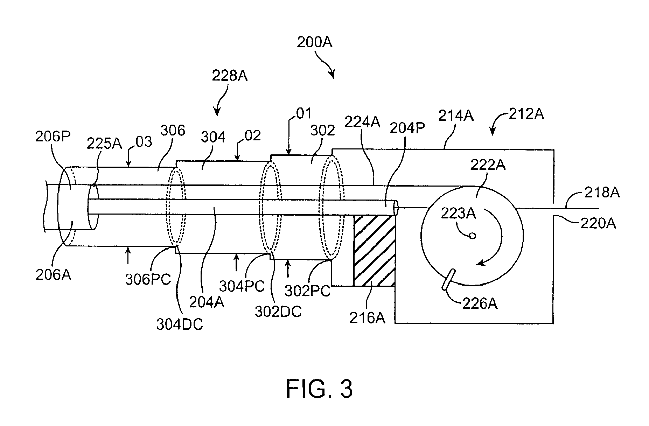

FIG. 3 is a partially cutaway delivery system 200A having a telescoping strain relief 228A in accordance with one embodiment of the present invention. Delivery system 200A of FIG. 3 is substantially similar to delivery system 200 of FIG. 2 and only the significant differences between delivery system 200A and delivery system 200 are discussed below. Specifically, pushrod 204A, sheath 206A, handle 212A, housing 214A, pushrod anchor 216A, guide wire 218A, guide wire port 220A, spool 222A, axle 223A, retraction wire 224A, bond 225A, knob 226A of delivery system 200A of FIG. 3 are similar to pushrod 204, sheath 206, handle 212, housing 214, pushrod anchor 216, guide wire 218, guide wire port 220, spool 222, axle 223, retraction wire 224, bond 225, knob 226 of delivery system 200 of FIG. 2, respectively, and so are not discussed further.

Telescoping strain relief 228A is telescoping and includes cylindrical sections 302, 304, 306 of decreasing diameter. Specifically, cylindrical section 302 adjacent handle 212A has the greatest diameter D1 of cylindrical sections 302, 304, 306. Conversely, cylindrical section 306 is furthest away from handle 212 and has the smallest diameter D3 of cylindrical sections 302, 304, 306. Cylindrical section 304 is between cylindrical sections 302, 306 and has a diameter D2 smaller than diameter D1 of cylindrical section 302 and larger than diameter D3 of cylindrical section 306.

Cylindrical sections 302, 304, 306 slide one within another to allow telescopic strain relief 228A to be telescoped to and from handle 212A. In the view of FIG. 3, telescopic strain relief 228A is fully extended, sometimes called telescoped from, handle 212A. For example, the physician grasps and pulls telescoping strain relief 228A from housing 214A thus telescoping and extending telescopic strain relief 228. When fully extended, telescopic strain relief 228A, and, more particularly, cylindrical section 306, extends over proximal end 206P of sheath 206A.

In the example shown in FIG. 3, cylindrical sections 302, 304, 306 include lips or other features to prevent cylindrical sections 302, 304, 306 from being separated from one another and from housing 214A. Specifically, cylindrical section 302 includes proximal and distal lips 302PL, 302DL, cylindrical section 304 includes proximal and distal lips 304PL, 304DL, and cylindrical section 306 includes a proximal lip 306PL.

Proximal lip 302PL of cylindrical section 302 catches on housing 214A preventing cylindrical section 302 from being separated from housing 214A. Proximal lip 304PL of cylindrical section 304 catches on distal lip 302DL of cylindrical section 302 preventing cylindrical section 304 from being separated from cylindrical section 302. Proximal lip 306PL of cylindrical section 306 catches on distal lip 304DL of cylindrical section 304 preventing cylindrical section 306 from being separated from cylindrical section 304.

Although use of cylindrical sections having lips to prevent the cylindrical sections from being separated from one another is discussed above and illustrated in FIG. 3, in other examples, other means are used to prevent the cylindrical sections from being separated from one another. For example, a friction fit between cylindrical sections is used to prevent the cylindrical sections from being separated one from another.

Further, although a telescoping strain relief having three cylindrical sections is discussed above and illustrated in FIG. 3, and other examples, telescoping strain reliefs are formed having more or less than three cylindrical sections, i.e., at least one cylindrical section. Further, the cylindrical sections may not be exactly cylindrical, but taper from the proximal end to the distal end in another example.

Numerous variations, whether explicitly provided for by the specification or implied by the specification or not, such as variations in structure, dimension, type ,of material and manufacturing process may be implemented by one of skill in the art in view of this disclosure.

## Claims

1. A stent delivery system (200) comprising:
a handle (212) comprising a housing (214) and a spool (222);
a pushrod (204) having a proximal end (204P) connected to said housing (214) of said handle (212);
a stent (202) located near a distal end (204D) of said pushrod (204), wherein said handle (212) has a linear length (Y) less than a linear length (X) of said stent (202);
a sheath (206) constraining said stent (202) at a distal end (206D) of said sheath (206); and
a retraction wire (224) connected to a proximal end (206P) of said sheath (206) and to said spool (222).

2. The stent delivery system of Claim 1 wherein rotation of said spool (222) coils said retraction wire (224) around said spool (222).

3. The stent delivery system of Claim 2 wherein coiling of said retraction wire (224) around said spool (222) retracts said sheath (206) deploying said stent (202).

4. The stent delivery system of any of the preceding claims wherein said stent (202) is a self-expanding stent, said stent (202) self-expanding upon retraction of said sheath (206).

5. The stent delivery system of any of the preceding claims further comprising a pushrod anchor (216) connecting said proximal end (204P) of said pushrod (204) to said housing (214).

6. The stent delivery system of any of the preceding claims wherein said spool (222) comprises a spool knob (226) for rotation of said spool (222).

7. The stent delivery system of any of the preceding claims wherein said pushrod (204) comprises a guide wire lumen, said stent delivery system further comprising a guide wire (218) extending through said pushrod (204).

8. The stent delivery system of Claim 7 wherein said housing (214) comprises a guide wire port (220), said guide wire (218) extending out a distal end (204D) of said pushrod (204), through said handle (212), and out said guide wire port (220).

9. The stent delivery system of any of the preceding claims wherein said sheath (206) comprises a pushrod lumen, said pushrod (204) extending through said sheath (206).

10. The stent delivery system of any of the preceding claims further comprising a bond (225) between said retraction wire (224) and said proximal end (206P) of said sheath (206).

11. The stent delivery system of any of the preceding claims further comprising a strain relief (228) coupled to said handle (212).

12. The stent delivery system of Claim 11 wherein said strain relief (228) extends a distance from said handle (212) sufficient to overlap said proximal end (206P) of said sheath (206).

13. The stent delivery system of Claim 11 or 12 wherein said proximal end (206P) of said sheath (206) is located inside of said strain relief (228).

14. The stent delivery system of any of Claims 11 to 13 wherein said strain relief (228A) is telescoping.

15. The stent delivery system of Claim 14 wherein said strain relief (228A) comprises at least one cylindrical section (302, 304, 306).

16. The stent delivery system of Claim 15 wherein said at least one cylindrical section comprises a first cylindrical section (302), said first cylindrical section (302) comprising a proximal lip (302PL), said proximal lip (302PL) of said first cylindrical section (302) catching on said housing (214A) to prevent said first cylindrical section (302) from being separated from said housing (214A).

17. The stent delivery system of Claim 16 wherein said at least one cylindrical section further comprises a second cylindrical section (304), said second cylindrical section (304) comprising a proximal lip (304PL), said proximal lip (304PL) of said second cylindrical section (304) catching on said first cylindrical section (302) to prevent said second cylindrical section (304) from being separated from said first cylindrical section (302).

18. The stent delivery system of any of Claims 14 to 17 wherein said strain relief (228A) is telescoped from said handle (212A).

19. A stent delivery system comprising:
a handle comprising a housing, a spool, and an axle mounting said spool to said housing;
a pushrod having a proximal end connected to said housing of said handle;
a stent located near a distal end of said pushrod, wherein said handle has a linear length less than a linear length of said stent;
a sheath constraining said stent at a distal end of said sheath;
a retraction wire connected to a proximal end of said sheath and to said spool; and
a telescoping strain relief coupled to said housing.

20. A stent delivery system comprising:
a handle comprising a housing;
a pushrod having a proximal end connected to said housing of said handle;
a stent located near a distal end of said pushrod, wherein said handle has a linear length less than a linear length of said stent;
a sheath constraining said stent at a distal end of said sheath; and
a retraction wire connected to a proximal end of said sheath and to a means for retracting said retraction wire.

21. The stent delivery system of Claim 20 wherein said means for retracting comprises a spool.
